# EUROPEAN PATENT SPECIFICATION

(11) **EP 1 756 066 B1**
(45) Date of publication and mention of the grant of the patent: **20.08.2008**
(21) Application number: 05752773.1
(22) Date of filing: 26.05.2005
(51) Int. Cl.: C07D 233/90, A61K 31/454, A61K 31/4164, A61P 25/00

(54) **TETRASUBSTITUTED IMIDAZOLE DERIVATIVES AS CANNABINOID CB1 RECEPTOR MODULATORS WITH A HIGH CB1/CB2 RECEPTOR SUBTYPE SELECTIVITY**
TETRASUBSTITUIERTE IMIDAZOLDERIVATE ALS CANNABINOID-CB1-REZEPTORMODULATOREN MIT HOHER CB1-/CB2-REZEPTORSUBTYP-SELEKTIVITÄT
DERIVES D'IMIDAZOLE TETRASUBSTITUES UTILISES COMME MODULATEURS DU RECEPTEUR DES CANNABINOIDES CB1 A ACTIVITE DE SELECTIVITE DE SOUS-TYPES DU RECEPTEUR CB1/CB2 ELEVEE

(30) Priority: 28.05.2004 US 574939 P; 28.05.2004 EP 04076619
(43) Date of publication of application: 28.02.2007
(73) Proprietor: Solvay Pharmaceuticals B.V., 1381 CP Weesp (NL)
(72) Inventor: LANGE, Josephus H.M., NL-1381 CP Weesp (NL); WALS, Henderik C., NL-1381 CP Weesp (NL); KRUSE, Cornelis G., NL-1381 CP Weesp (NL)
(74) Representative: Verhage, Marinus
(86) International application number: PCT/EP2005/052405
(87) International publication number: WO 2005/118553

(56) References cited:
- WO-A-03/027076
- LANGE J H M ET AL: "SYNTHESIS, BIOLOGICAL PROPERTIES, AND MOLECULAR MODELING INVESTIGATIONS OF NOVEL 3,4-DIARYLPYRAZOLINES AS POTENT AND SELECTIVE CB1 CANNABINOID RECEPTOR ANTAGONISTS" JOURNAL OF MEDICINAL CHEMISTRY, AMERICAN CHEMICAL SOCIETY, US, vol. 47, no. 3, 2004, pages 627-643, XP001188902 ISSN: 0022-2623
- DYCK B ET AL: "POTENT IMIDAZOLE AND TRIAZOLE CB1 RECEPTOR ANTAGONISTS RELATED TO SR141716" BIOORGANIC & MEDICINAL CHEMISTRY LETTERS, OXFORD, GB, vol. 14, 2004, pages 1151-1154, XP001197288 ISSN: 0960-894X cited in the application

## Description

The present invention relates to 1,2,4,5-tetrasubstituted imidazole derivatives as selective cannabinoid CB₁ receptor modulators, in particular CB₁ receptor antagonists or inverse agonists having a high CB₁/CB₂ receptor subtype selectivity, to methods for the preparation of these compounds and to novel intermediates useful for the synthesis of said imidazole derivatives. The invention also relates to the use of a compound disclosed herein for the manufacture of a medicament giving a beneficial effect. A beneficial effect is disclosed herein or apparent to a person skilled in the art from the specification and general knowledge in the art. The invention also relates to the use of a compound of the invention for the manufacture of a medicament for treating or preventing a disease or condition. More particularly, the invention relates to a new use for the treatment of a disease or condition disclosed herein or apparent to a person skilled in the art from the specification and general knowledge in the art. In embodiments of the invention specific compounds disclosed herein are used for the manufacture of a medicament useful in the treatment of psychiatric and neurological disorders.

Multisubstituted imidazole derivatives having CB₁ receptor affinity are known from WO 03/027076 and WO 03/063781. Furthermore, WO 03/040107 disclosed imidazoles for the treatment of obesity. In addition, an article has been published wherein imidazoles are described as CB₁ receptor antagonists (Dyck et al., Bioorg. Med. Chem. Lett. 2004, 14, 1151-1154). The patent applications and the article mentioned above do not disclose data on CB₁/CB₂ receptor subtype selectivity of the disclosed compounds therein.

CB₁ receptor modulators have several potential applications such as medicaments for treating psychosis, anxiety, depression, attention deficits, memory disorders, cognitive disorders, appetite disorders, obesity, addiction, appetence, drug dependence, neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, septic shock, glaucoma, diabetes, cancer, emesis, nausea, gastrointestinal disorders, gastric ulcers, diarrhoea, sexual disorders, impulse control disorders and cardiovascular disorders.

CB₂ receptors occur predominantly in the immune system (spleen, tonsils, immune cells) as well as in microglial cells and astrocytes and have been linked to the perception of neuropathic pain. Potent CB₁ receptor modulators having low CB₂ receptor affinity (i.e. compounds having a high CB₁/CB₂ receptor subtype selectivity) are advantageous compounds as compared to non-selective or less selective cannabinoid receptor modulators as they will be devoid of undesired potential CB₂ receptor-mediated side-effects, such as immunologic side-effects or inflammatory related side-effects or effects on neuropathic pain perception.

The objective of the present invention was to develop imidazole derivatives with a high CB₁/CB₂ receptor subtype selectivity.

Surprisingly, we have found that the modification of the original CH₂ group X in prior art imidazoles of general formula (I) by a moiety containing a sulphur atom results in novel compounds with CB₁/CB₂ receptor subtype selectivities enhanced by an ample factor 10, thus resulting in CB₁/CB₂ affinity ratio's of well over 100. Compounds of the general formula (I) : 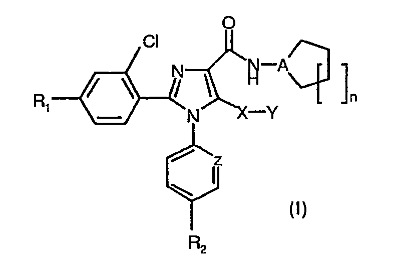 wherein
- R₁ represents a chloro, bromo, fluoro or hydrogen atom,
- R₂ represents a chloro or bromo atom, or a CF₃ group,
- A represents a nitrogen atom or a CH group,
- X represents a sulphur atom or a sulfoxide (S=O) moiety or a sulfone (SO₂) moiety,
- Y represents a hydrogen atom or a methyl, ethyl, n-propyl, isopropyl, n-butyl, t-butyl, trifluoromethyl, phenyl, benzyl or pyridyl group,
- Z represents a nitrogen atom or a CH group,
- n represents the value 1, 2 or 3,
and tautomers, stereoisomers, prodrugs and pharmacologically acceptable salts thereof are new and are potent and CB₁/CB₂ selective CB₁ receptor antagonists or inverse agonists.

All sulfoxides within this invention contain a centre of chirality. The invention relates to racemates, mixtures of diastereomers as well as the individual stereoisomers of the compounds having formula (I). The invention also relates to the E isomer, Z isomer and E/Z mixtures of compounds having formula (I).

The invention particularly relates to compounds of the general formula (I) in which R₁ represents a hydrogen atom or a chloro atom and R₂ is a chloro atom, Y represents a methyl or ethyl group, Z is CH, n represents 1, 2 or 3, A and X have the meanings as given above, and tautomers, stereoisomers, prodrugs and pharmacologically acceptable salts thereof.

More in particular the invention relates to compounds of general formula (I) in which R₁ and R₂ represent chloro atoms, Y represents a methyl group, Z is CH, n represents 1, 2 or 3, A is a nitrogen atom and X has the meanings as given above, and tautomers, stereoisomers, prodrugs and pharmacologically acceptable salts thereof.

### GENERAL ASPECTS OF SYNTHESES

The synthesis of compounds having formula (I) is outlined in Scheme 1. The carboxylic acid of general formula (II) can be converted to the corresponding tert-butyl ester (III). This ester (III) can be treated with a strong non-nucleophilic base in an inert anhydrous organic solvent and subsequently reacted with a sulphur-derived electrophile YSSY wherein Y represents a methyl, ethyl, n-propyl, isopropyl, n-butyl, t-butyl, trifluoromethyl, phenyl, benzyl or pyridyl group to afford a compound of general formula (IV). This compound of general formula (IV) can be oxidised with one molar equivalent of meta-chloroperbenzoic acid to give the corresponding sulfoxide analogue. Alternatively, reaction of a compound of general formula (IV) with two molar equivalents of meta-chloroperbenzoic acid can convert the sulphur moiety to the corresponding sulfone moiety. The ester of general formula (IV) can be hydrolysed - preferably under acidic conditions - to give the corresponding carboxylic acid (V). The resulting compound of general formula (V) can be coupled with an amine in the presence of an activating or coupling reagent to give a compound of general formula (I), wherein R_{1,} R_{2,} A, X, Y, Z and n have the abovementioned meaning.

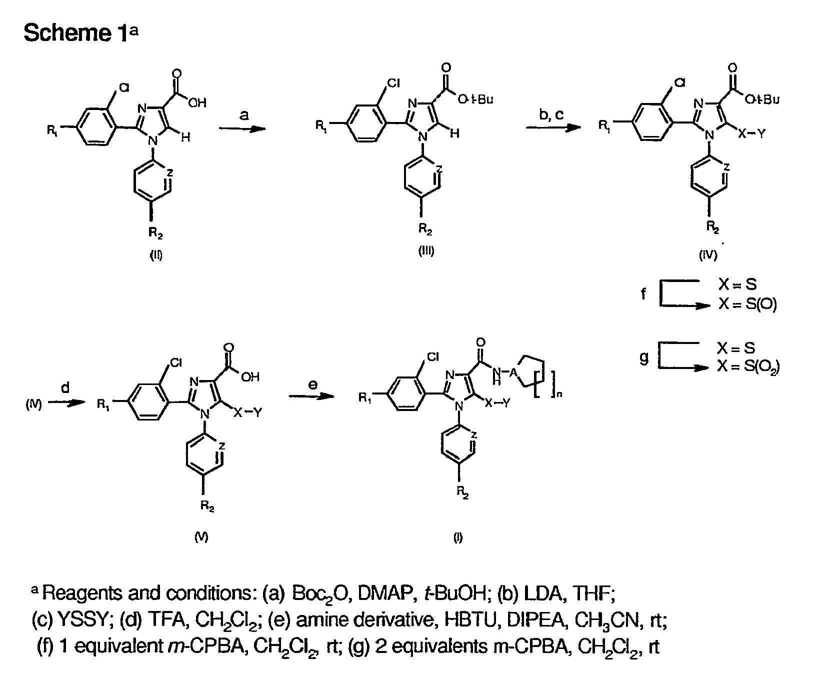

Alternatively, a compound of general formula (VI) wherein R_{1,} R_{2,} A, Z and n have the abovementioned meaning can be reacted with a strong non-nucleophilic base, such as LDA, in an inert anhydrous organic solvent, followed by treatment with sulphur (S₈) or with a sulphur-derived electrophile YSSY wherein Y represents a methyl, ethyl, propyl, isopropyl, n-butyl, t-butyl, trifluoromethyl, phenyl, benzyl or pyridyl group to give a compound of general formula (I), wherein X represents a sulphur atom, Y represents a hydrogen atom or a methyl, ethyl, n-propyl, isopropyl, n-butyl, t-butyl, trifluoromethyl, phenyl, benzyl or pyridyl group and wherein R_{1,} R_{2,} A, Z and n have the abovementioned meaning (Scheme 2).

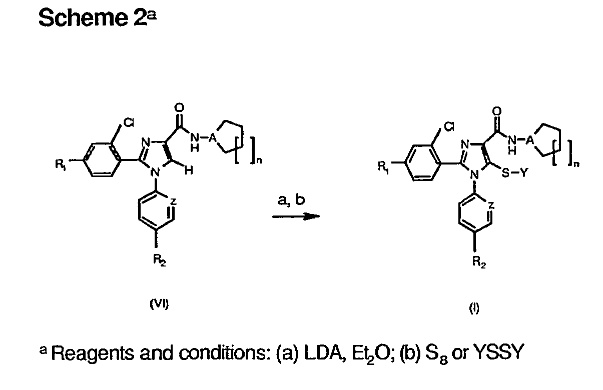

Additional information on activating and coupling methods of amines to carboxylic acids can be found in:
a) M. Bodanszky and A. Bodanszky: The Practice of Peptide Synthesis, Springer-Verlag, New York, 1994; ISBN: 0-387-57505-7;
b) K. Akaji et al., Tetrahedron Lett. (1994), 35, 3315-3318);
c) F. Albericio et al., Tetrahedron Lett. (1997), 38, 4853-4856).

The selection of the particular synthetic procedures depends on factors known to those skilled in the art such as the compatibility of functional groups with the reagents used, the possibility to use protecting groups, catalysts, activating and coupling reagents and the ultimate structural features present in the final compound being prepared.

Pharmaceutically acceptable salts may be obtained using standard procedures well known in the art, for example by mixing a compound of the present invention with a suitable acid, for instance an inorganic acid or an organic acid.

### PHARMACEUTICAL PREPARATIONS

The compounds of the invention can be brought into forms suitable for administration by means of usual processes using auxiliary substances such as liquid or solid carrier material. The pharmaceutical compositions of the invention may be administered enterally, orally, parenterally (intramuscularly or intravenously), rectally or locally (topically). They can be administered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer substances. Frequently used auxiliary substances which may be mentioned are magnesium carbonate, titanium dioxide, lactose, mannitol and other sugars or sugar alcohols, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Compounds of the present invention are generally administered as pharmaceutical compositions which are important and novel embodiments of the invention because of the presence of the compounds, more particularly specific compounds disclosed herein. Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge in the art. The invention also includes the preparation or manufacture of said pharmaceutical compositions.
In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

Due to the potent CB₁ antagonistic or inverse agonist activity the compounds according to the invention are suitable for use in the treatment of psychiatric disorders such as psychosis, anxiety, depression, attention deficits, memory disorders, cognitive disorders, appetite disorders, obesity, in particular juvenile obesity and drug induced obesity, addiction, appetence, drug dependence and neurological disorders such as neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, and other diseases involving cannabinoid neurotransmission, including the treatment of septic shock, glaucoma, cancer, diabetes, emesis, nausea, asthma, respiratory diseases, gastrointestinal disorders, gastric ulcers, diarrhoea, sexual disorders, impulse control disorders and cardiovascular disorders.
The cannabinoid receptor modulating activity of the compounds of the invention makes them particularly useful in the treatment of obesity, juvenile obesity and drug induced obesity, especially when used in combination with lipase inhibitors. Specific examples of compounds which can be used in such combination preparations are (but not restricted to) the synthetic lipase inhibitor orlistat, lipase inhibitors isolated from micro organisms such as lipstatin (from *Streptomyces toxytricini*), ebelactone B (from *Streptomyces aburaviensis*), synthetic derivatives of these compounds, as well as extracts of plants known to possess lipase inhibitory activity, for instance extracts of *Alpinia officinarum* or compounds isolated from such extracts like 3-methylethergalangin (from *A. officinarum*).

### PHARMACOLOGICAL METHODS

### In vitro affinity for cannabinoid-CB₁ receptors

The affinity of the compounds of the invention for cannabinoid CB₁ receptors can be determined using membrane preparations of Chinese hamster ovary (CHO) cells in which the human cannabinoid CB₁ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro affinity for cannabinoid-CB₂ receptors

The affinity of the compounds of the invention for cannabinoid CB₂ receptors can be determined using membrane preparations of Chinese hamster ovary (CHO) cells in which the human cannabinoid CB₂ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro cannabinoid-CB₁ receptor antagonism

In vitro CB₁ receptor antagonism can be assessed with the human CB₁ receptor cloned in Chinese hamster ovary (CHO) cells. CHO cells are grown in a Dulbecco's Modified Eagle's medium (DMEM) culture medium, supplemented with 10% heat-inactivated fetal calf serum. Medium is aspirated and replaced by DMEM, without fetal calf serum, but containing [³H]-arachidonic acid and incubated overnight in a cell culture stove (5% CO₂/95% air; 37 ºC; water-saturated atmosphere). During this period [³H]-arachidonic acid is incorporated in membrane phospholipids. On the test day, medium is aspirated and cells are washed three times using 0.5 ml DMEM, containing 0.2% bovine serum albumin (BSA). Stimulation of the CB₁ receptor by WIN 55,212-2 leads to activation of PLA₂ followed by release of [³H]-arachidonic acid into the medium. This WIN 55,212-2-induced release is concentration-dependently antagonized by CB₁ receptor antagonists.

### DOSE

The affinity of the compounds of the invention for CB₁ receptors was determined as described above. From the binding affinity measured for a given compound of formula (1), one can estimate a theoretical lowest effective dose. At a concentration of the compound equal to twice the measured K-value, nearly 100% of the CB₁ receptors likely will be occupied by the compound. Converting that concentration to mg of compound per kg of patient yields a theoretical lowest effective dose, assuming ideal bioavailability. Pharmacokinetic, pharmacodynamic, and other considerations may alter the dose actually administered to a higher or lower value. The dosage expediently administered is 0.001 - 1000 mg/kg, preferably 0.1-100 mg/kg of patient's bodyweight.

### EXAMPLES

### EXAMPLE 1: MATERIALS AND METHODS

¹H and ¹³C NMR spectra were recorded on a Bruker Avance DRX600 instrument (600 MHz), Varian UN400 instrument (400 MHz) or on a Varian VXR200 instrument (200 MHz) using DMSO-*d*₆ or CDCl₃ as solvents with tetramethylsilane as an internal standard. Chemical shifts are given in ppm (δ scale) downfield from tetramethylsilane. Coupling constants (*J*) are expressed in Hz. Flash chromatography was performed using silica gel 60 (0.040-0.063 mm, Merck). Column chromatography was performed using silica gel 60 (0.063-0.200 mm, Merck). Melting points were recorded on a Büchi B-545 melting point apparatus. Mass spectra were recorded on a Micromass QTOF-2 instrument with MassLynx application software for acquisition and reconstruction of the data. Exact mass measurement was done of the quasimolecular ion [M+H]⁺.

### EXAMPLE 2: SYNTHESES OF SPECIFIC COMPOUNDS

### Compounds 1 - 3

**1-(4-Chlorophenyl)-2-(2,4-dichlorophenyl)-1*H*-imidazole-4-carboxylic acid**
To a magnetically stirred solution of ethyl 1-(4-chlorophenyl)-2-(2,4-dichlorophenyl)-1 H-imidazole-4-carboxylate (18.44 g, 0.0466 mol) in THF (240 ml) was added LiOH (2.24 g, 0.0932 mol) and H₂O (240 ml). The resulting mixture was stirred at 50 °C for 16 h to give a clear solution. After cooling to room temperature, HCl (1 N solution, 95 ml) and H₂O (240 ml) were added to give a precipitate which was collected by filtration, washed with water and dried *in vacuo* to give 1-(4-chlorophenyl)-2-(2,4-dichlorophenyl)-1*H*-imidazole-4-carboxylic acid (16.83 g, 98 % yield), mp 138-142 °C (decomposition); ¹H-NMR (600 MHz, DMSO-d₆) δ 7.08 (br d, *J* = 8 Hz, 2H), 7.31-7.37 (m, 4H), 7.45 (d, *J* = 8 Hz, 1H), 7.96 (s, 1H); ¹³C-NMR (150 MHz, DMSO-d₆) δ 126.87, 127.85, 127.91, 128.47, 129.36, 129.66, 133.56, 133.99, 134.44, 134.49, 135.54, 135.99, 143.77, 163.67.

***Tert*-butyl 1-(4-chlorophenyl)-2-(2,4-dichlorophenyl)-1*H*-imidazole-4-carboxylate**
To a magnetically stirred mixture of 1-(4-chlorophenyl)-2-(2,4-dichlorophenyl)-1*H-*imidazole-4-carboxylic acid (20.77 g, 0.0565 mol) and Boc₂O (24.63 g, 0.113 mol) in *t*-BuOH (275 ml) was added DMAP (2.07 g, 0.017 mol) and the resulting mixture was stirred for 16 h. After concentration in vacuo, toluene was added and the mixture was again concentrated. The residue was purified by column chromatography (CH₂Cl₂/acetone = 95/5 (v/v)) and recrystallized from diisopropyl ether to give *tert-*butyl 1-(4-chlorophenyl)-2-(2,4-dichlorophenyl)-1*H*-imidazole-4-carboxylate (15.75 g, 66 % yield), mp 178-180 °C; ¹H-NMR (200 MHz, CDCl₃) δ 1.63 (s, 9H), 7.05 (brd, *J* ∼ 8 Hz, 2H), 7.25-7.37 (m, 4H), 7.52 (d, *J* = 8 Hz, 1H), 7.80 (s, 1H).

***Tert*-butyl 1-(4-chlorophenyl)-2-(2,4-dichlorophenyl)-5-methylsulfanyl-1*H*-imidazole-4-carboxylate.** To a cooled (-20 °C) and magnetically stirred solution of *tert-*butyl 1-(4-chlorophenyl)-2-(2,4-dichlorophenyl)-1*H*-imidazole-4-carboxylate (10.59 g, 25.0 mmol), in anhydrous THF (100 ml) was added LDA (15.0 ml, 2 M solution in heptane/THF, 30.0 mmol) and the resulting mixture was stirred for 1 hour under N₂. A solution of (CH₃S)₂ (2.7 ml, 30.0 mmol) in THF (20 ml) was added and the resulting solution was successively stirred at -40 °C for 1 h, allowed to attain room temperature and stirred for another 16 h. A saturated aqueous NH₄Cl solution (250 ml) was added and the resulting solution was extracted twice with ethyl acetate (EtOAc). The combined organic layers were washed with water, dried over MgSO₄, filtered and concentrated to give *tert*-butyl 1-(4-chlorophenyl)-2-(2,4-dichlorophenyl)-5-methylsulfanyl-1*H*-imidazole-4-carboxylate in 90 % yield as an oil which slowly solidified; ¹H-NMR (200 MHz, CDCl₃) δ 1.66 (s, 9H), 2.28 (s, 3H), 7.05 (br d, *J* ∼ 8 Hz, 2H), 7.25 (dd, *J* = 8 and 2 Hz, 1H), 7.28 (d, *J* = 2 Hz, 1H), 7.32-7.41 (m, 3H).

***Tert*-butyl 1-(4-chlorophenyl)-2-(2,4-dichlorophenyl)-5-methylsulfonyl-1*H*-imidazole-4-carboxylate.** To a magnetically stirred solution of *tert*-butyl 1-(4-chlorophenyl)-2-(2,4-dichlorophenyl)-5-methylsulfanyl-1*H*-imidazole-4-carboxylate (6.00 g, 12.8 mmol) in CH₂Cl₂ (25 ml) was slowly added a solution of *m*-CPBA (6.90 g, 70 % grade, 0.282 mol) in CH₂Cl₂ and the resulting mixture was stirred for 16 hours. The reaction mixture was twice washed with 2N NaOH solution and dried over Na₂SO₄, filtered and concentrated. The residue was purified by flash chromatography (Et₂O/ petroleum ether = 2/1 (v/v)) to give *tert*-butyl 1-(4-chlorophenyl)-2-(2,4-dichlorophenyl)-5-methylsulfonyl-1*H*-imidazole-4-carboxylate (4.76 g, 74 % yield) as a white solid, mp 130 °C; ¹H-NMR (400 MHz, CDCl₃) δ 1.66 (s, 9H), 3.34 (s, 3H), 7.15 (br d, *J* ∼ 8 Hz, 2H), 7.20-7.26 (m, 2H), 7.32-7.41 (m, 3H).
Analogously was prepared *tert*-butyl 1-(4-chlorophenyl)-2-(2,4-dichlorophenyl)-5-methylsulfinyl-1*H*-imidazole-4-carboxylate by performing the reaction with 1 molar equivalent of *m*-CPBA instead of two molar equivalents; ¹H-NMR (400 MHz, CDCl₃) δ 1.64 (s, 9H), 2.94 (s, 3H), 7.20-7.36 (m, 7H).

**1-(4-Chlorophenyl)-2-(2,4-dichlorophenyl)-5-methylsulfonyl-1*H*-imidazole-4-carboxylic acid.** To a magnetically stirred solution of *tert*-butyl 1-(4-chlorophenyl)-2-(2,4-dichlorophenyl)-5-methylsulfonyl-1*H*-imidazole-4-carboxy-late (4.76 g, 9.49 mmol) in CH₂Cl₂ (60 ml) was added excess TFA (9.40 ml, 0.2124 mol) and Et₃SiH (3.8 ml, 0.0238 mol). The solution was reacted at room temperature for 16 h and concentrated in vacuo. Water was added and the formed precipitate was collected by filtration and subsequently dried to give 1-(4-chlorophenyl)-2-(2,4-dichlorophenyl)-5-methylsulfonyl-1*H*-imidazole-4-car-boxylic acid in quantitative yield, mp ∼ 130°C (dec); ¹H-NMR (400 MHz, CDCl₃) δ 3.45 (s, 3H), 3.50 (br s, 1H), 7.40 (br d, *J* ∼ 8 Hz, 2H), 7.42 (dd, *J* = 8 and 2 Hz, 1H), 7.50 (br d, *J* ∼8 Hz, 2H), 7.59 (d, *J* = 2 Hz, 1 H), 7.61 (d, *J* = 8 Hz, 1H).
Analogously was prepared 1-(4-chlorophenyl)-2-(2,4-dichlorophenyl)-5-methylsulfinyl-1*H*-imidazole-4-carboxylic acid ¹H-NMR (400 MHz, CDCl₃) δ 2.99 (s, 3H), 7.37-7.60 (m, 7H), 13.20 (br s, 1H).

**1-(4-Chlorophenyl)-2-(2,4-dichlorophenyl)-5-methylsulfonyl-*N*-(piperidin-1-yl)-1*H*-imidazole-4-carboxamide (compound 1).**
To a magnetically stirred suspension of 1-(4-chlorophenyl)-2-(2,4-dichlorophenyl)-5-methylsulfonyl-1*H*-imidazole-4-carboxylic acid (2.23 g, 5.01 mmol) in anhydrous CH₃CN (50 ml) was successively added *N,N*-diisopropylethylamine (Hunig's base) (1.90 ml, 11.0 mmol), O-benzotriazol-1-yl-*N, N, N', N'*-tetramethyluronium hexafluorophosphate (HBTU) (2.27 g, 5.99 mmol) and 1-aminopiperidine (0.65 ml, 6.03 mmol). After stirring for 16 h, the resulting mixture was concentrated in vacuo. The residue was dissolved in EtOAc, successively washed with aqueous NaHCO₃ solution, water and brine, dried over Na₂SO₄, filtered and concentrated to give a crude solid. This solid was further purified by flash chromatography (silicagel, EtOAc) and triturated with methyl-tert-butyl ether to give 1-(4-chlorophenyl)-2-(2,4-dichlorophenyl)-5-methylsulfonyl-*N*-(piperidin-1-yl)-1*H*-imidazole-4-carboxamide in 84 % yield, mp 181-185 °C (dec); ¹H-NMR (600 MHz, DMSO-d₆) δ 1.35-1.41 (m, 2H), 1.61-1.66 (m, 4H), 2.80-2.84 (m, 4H), 3.52 (s, 3H), 7.38 (d, *J* = 8 Hz, 2H), 7.42 (dd, *J* = 8 and 2 Hz, 1H), 7.46 (d, *J* = 8 Hz, 2H), 7.57 (d, *J* = 2 Hz, 1H), 7.62 (d, *J* = 8 Hz, 1H), 9.40 (s, 1H); HRMS (C₂₂H₂₂Cl₃N₄O₃S) [M+H]⁺: found *m*/*z* 527.0469, calcd 527.0478.

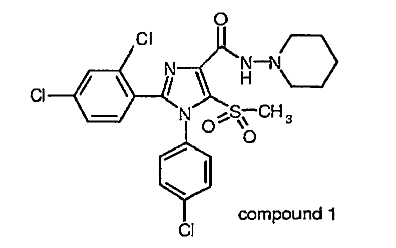

Analogously was prepared: 1-(4-chlorophenyl)-2-(2,4-dichlorophenyl)-5-methyl sulfonyl-*N*-(cyclohexyl)-1*H*-imidazole-4-carboxamide. **(compound 2)** Melting point: 191-192°C.

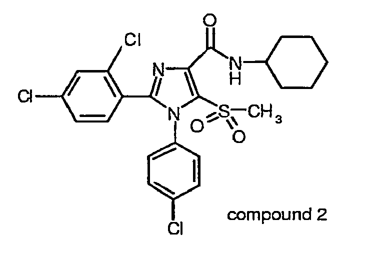

Analogously was prepared: 1-(4-chlorophenyl)-2-(2,4-dichlorophenyl)-5-methyl sulfinyl-*N*-(piperidin-1-yl)-1*H*-imidazole-4-carboxamide. **(compound 3)** Melting point: 218-221 °C.

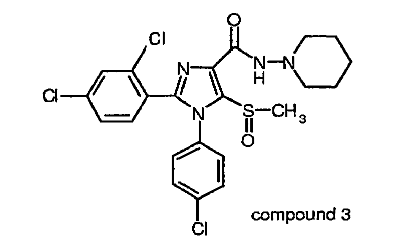

### Compounds 4 - 5

Racemic 1-(4-chlorophenyl)-2-(2,4-dichlorophenyl)-5-methylsulfinyl-*N*-(piperi-din-1-yl)-1*H*-imidazole-4-carboxamide (compound 3, 3.86 gram, 0.0075 mol) was separated into its enantiomers (-)-1-(4-chlorophenyl)-2-(2,4-dichloro-phenyl)-5-methylsulfinyl-*N*-(piperidin-1-yl)-1*H*-imidazole-4-carboxamide (**compound 4**, 1.3 gram) ([a_{D}25] = -19 °, c = 0.94 (g/100 ml solvent), methanol; enantiomeric excess 97.2 %: Melting point: 242-244 °C) and (+)-1-(4-chlorophenyl)-2-(2,4-dichlorophenyl)-5-methylsulfinyl-*N*-(piperidin-1-yl)-1*H*-imidazole-4-carboxamide (**compound 5,** 1.4 gram) ([a_{D}25] = +23 °, c = 0.94 (g/100 ml solvent), methanol: enantiomeric excess 99.5 %: Melting point: 243-245 °C), respectively by using preparative HPLC and a Chiralpak AD 20 µm chiral stationary phase. The mobile phase consisted of a mixture of 25 % ethanol/heptane (25/75 (v/v)).

### Compounds 6-11

**1-(4-Chlorophenyl)-2-(2,4-dichlorophenyl)-5-methylsulfanyl-1*H*-imidazole-4-carboxylic acid.** To a magnetically stirred solution of *tert*-butyl 1-(4-chlorophenyl)-2-(2,4-dichlorophenyl)-5-methylsulfanyl-1*H*-imidazole-4-carboxylate (4.00 g, 8.53 mmol) in CH₂Cl₂ (60 ml) was added excess TFA (8.40 ml, 0.111 mol). The solution was reacted at room temperature for 16 hours and subsequently concentrated in vacuo. Water was added and the formed precipitate was collected by filtration and subsequently dried to give 1-(4-chlorophenyl)-2-(2,4-dichlorophenyl)-5-methylsulfanyl-1*H*-imidazole-4-carboxylic acid in 98 % yield, mp ∼ 100 °C (decomposition); ¹H-NMR (400 MHz, CDCl₃) δ 2.41 (s, 3H), 3.60 (br s, 1H), 7.08 (br d, *J* ∼ 8 Hz, 2H), 7.26 (dd, *J* = 8 and 2 Hz, 1H), 7.30 (d, *J* = 8 Hz, 1H), 7.35 (d, *J* = 2 Hz, 1H), 7.37 (br d, *J* ∼ 8 Hz, 2H).

**1-(4-Chlorophenyl)-2-(2,4-dichlorophenyl)-5-methylsulfanyl-*N*-(piperidin-1-yl)-1*H*-imidazole-4-carboxamide (compound 6).** To a magnetically stirred suspension of 1-(4-chlorophenyl)-2-(2,4-dichlorophenyl)-5-methylsulfanyl-1*H*-imidazole-4-carboxylic acid (1.72 g, 4.16 mmol) in anhydrous CH₃CN (45 ml) was successively added *N,N*-diisopropylethylamine (Hunig's base) (1.60 ml, 9.20 mmol), O-benzotriazol-1-yl-*N, N, N', N'*-tetramethyluronium hexafluorophosphate (HBTU) (1.89 g, 4.99 mmol) and 1-aminopiperidine (0.54 ml, 5.01 mmol). After stirring for 40 h, water was added and the resulting mixture was extracted with dichloromethane. The dichloromethane layer was successively twice washed with an 1 N HCl solution and water, dried over MgSO₄, filtered and concentrated to give a crude oil. This oil was further purified by flash chromatography (silicagel, EtOAc) and triturated with diethyl ether to give 1-(4-chlorophenyl)-2-(2,4-dichlorophenyl)-5-methylsulfanyl-N-(piperidin-1-yl)-1*H*-imidazole-4-carboxamide in 72 % yield, mp 170 °C (dec); ¹H-NMR (600 MHz, DMSO-d₆) δ 1.35-1.42 (m, 2H), 1.62-1.67 (m, 4H), 2.35 (s, 3H), 2.80-2.84 (m, 4H), 7.29 (d, *J* = 8 Hz, 2H), 7.42 (dd, *J* = 8 and 2 Hz, 1H), 7.45 (d, *J* = 8 Hz, 2H), 7.52 (d, *J* = 2 Hz, 1H), 7.62 (d, *J* = 8 Hz, 1H), 8.90 (s, 1H); ¹³C-NMR (150 MHz, DMSO-d₆) δ 19.26, 23.32, 25.63, 55.98, 127.52, 128.61, 129.14, 129.23, 129.86, 130.12, 130.18, 133.86, 134.45, 134.66, 136.01, 137.12, 144.04, 158.98; HRMS (C₂₂H₂₂Cl₃N₄OS) [M+H]⁺: found *m*/*z* 495.0592, calcd 495.0580.

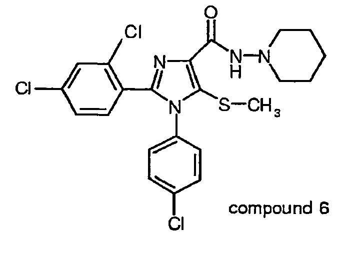

Analogously was prepared: 1-(4-chlorophenyl)-2-(2,4-dichlorophenyl)-5-methylsulfanyl-*N*-(cyclohexyl)-1*H*-imidazole-4-carboxamide. **(compound 7)** Melting point: 152-154 °C.

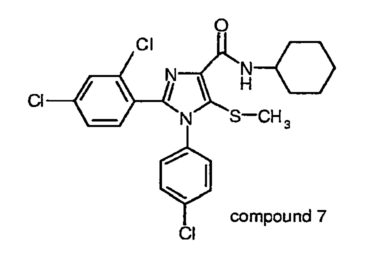

Analogously was prepared: 1-(4-chlorophenyl)-2-(2,4-dichlorophenyl)-5-ethylsulfanyl-*N*-(piperidin-1-yl)-1*H*-imidazole-4-carboxamide **(compound 8).** ¹H-NMR (400 MHz, CDCl₃) δ 1.09 (t, *J* = 7 Hz, 3H), 1.40-1.48 (m, 2H), 1.72-1.80 (m, 4H), 2.84-2.92 (m, 4H), 3.00 (q, *J* = 7 Hz, 2H), 7.03 (dt, *J* = 8 and 2 Hz, 2H), 7.23-7.35 (m, 5H), 8.02 (br s, 1H).

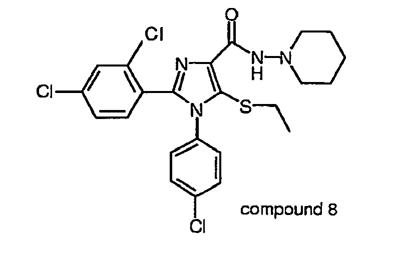

Analogously was prepared: 1-(4-chlorophenyl)-2-(2,4-dichlorophenyl)-5-methylsulfanyl-*N*-(pyrrolidin-1-yl)-1*H*-imidazole-4-carboxamide. **(compound 9)** Melting point: 158 °C.

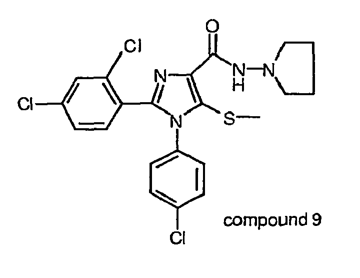

Analogously was prepared: 1-(4-chlorophenyl)-2-(2,4-dichlorophenyl)-5-methylsulfanyl-*N*-(azepan-1-yl)-1*H*-imidazole-4-carboxamide **(compound 10)** ¹H-NMR (400 MHz, CDCl₃) δ 1.63-1.68 (m, 4H), 1.74-1.81 (m, 4H), 2.42 (s, 3H), 3.17-3.22 (m, 4H), 7.04 (dt, *J* = 8 and 2 Hz, 2H), 7.23-7.36 (m, 5H), 8.50 (br s, 1H).

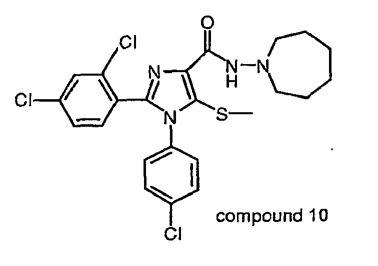

Analogously was prepared: 1-(4-chlorophenyl)-2-(2-chlorophenyl)-5-methylsulfanyl-*N*-(piperidin-1-yl)-1*H*-imidazole-4-carboxamide **(compound 11)** Melting point: 192-193 °C.

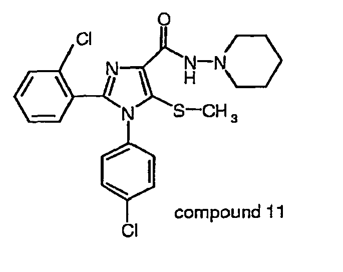

The specific compounds of which the synthesis is described above are intended to further illustrate the invention in more detail, and therefore are not deemed to restrict the scope of the invention in any way. Other embodiments of the invention will be apparent to those skilled in the art from consideration of the specification and practice of the invention disclosed herein. It is thus intended that the specification and examples be considered as exemplary only.

### EXAMPLE 4: FORMULATIONS USED IN ANIMAL STUDIES

**For oral *(p.o.)* administration**: to the desired quantity (0.5-5 mg) of the solid compound 1 in a glass tube, some glass beads were added and the solid was milled by vortexing for 2 minutes. After addition of 1 ml of a solution of 1% methylcellulose in water and 2% (v/v) of Poloxamer 188 (Lutrol F68), the compound was suspended by vortexing for 10 minutes. The pH was adjusted to 7 with a few drops of aqueous NaOH (0.1N). Remaining particles in the suspension were further suspended by using an ultrasonic bath.

**For intraperitoneal *(i.p.)* administration**: to the desired quantity (0.5-15 mg) of the solid compound 1 in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE 5: PHARMACOLOGICAL TESTRESULTS

Some CB₁/CB₂ receptor affinity data (mean results of at least three independent experiments, performed according to the protocols given above) of prior art compounds and representative compounds of this invention are shown in the table below. These data illustrate the impact on CB_{1/2} receptor selectivity ratios achieved by the structural modification that forms the basis of the present invention: the new compounds retain their high affinity for the cannabinoid-CB₁ receptor, whilst that for the CB₂ receptor is very substantially reduced.

The compounds in Table 1 have the following general structural formula:

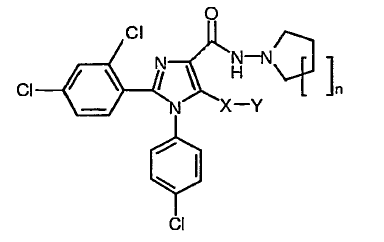

**Table 1. CB₁ and CB₂ receptor affinities of prior art compounds (entries 1 and 2) and representative compounds of this invention (entries 3 - 6).**

| **Entry** | **X** | **Y** | **n** | **CB₁ (nM)** | **CB₂ (nM)** | **CB₁/CB₂ ratio** |
|---|---|---|---|---|---|---|
| | | | | | | |
| prior art | CH₂ | H | 2 | 30 | 608 | **20** |
| prior art | CH₂ | CH₃ | 2 | 14 | 430 | **31** |
| | | | | | | |
| compound 6 | S | CH₃ | 2 | 12 | 2,057 | **171** |
| compound 1 | SO₂ | CH₃ | 2 | 12 | 7,652 | **638** |
| compound 9 | S | CH₃ | 1 | 5 | > 1,000 | **> 200** |
| compound 10 | S | CH₃ | 3 | 7 | > 1,000 | **> 142** |

## Claims

1. Compounds of the general formula (I) 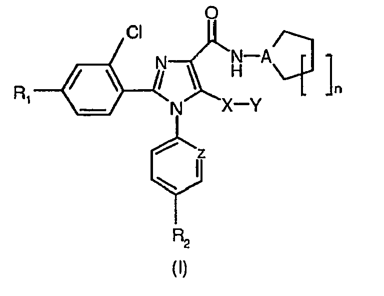 wherein
- R₁ represents a chloro, bromo, fluoro or hydrogen atom,
- R₂ represents a chloro or a bromo atom, or a CF₃ group,
- A represents a nitrogen atom or a CH group,
- X represents a sulphur atom or a sulfoxide (S=O) moiety or a sulfone (SO₂) moiety,
- Y represents a hydrogen atom or a methyl, ethyl, n-propyl, isopropyl, n-butyl, t-butyl, trifluoromethyl, phenyl, benzyl or pyridyl group,
- Z represents a nitrogen atom or a CH group,
- n represents the value 1, 2 or 3,
and tautomers, stereoisomers and pharmacologically acceptable salts thereof.

2. Compounds as claimed in claim 1 of general formula (I) in which R₁ represents a hydrogen atom or a chloro atom, R₂ is a chloro atom, Y represents a methyl or ethyl group, Z is CH, n represents 1, 2 or 3, A and X have the meanings as given in claim 1, and tautomers, stereoisomers and pharmacologically acceptable salts thereof.

3. Compound as claimed in claim 2 of general formula (I) in which R₁ represents a chloro atom, Y represents a methyl group, A is a nitrogen atom and tautomers, stereoisomers and pharmacologically acceptable salts thereof.

4. Pharmaceutical compositions comprising, in addition to a pharmaceutically acceptable carrier and/or at least one pharmaceutically acceptable auxiliary substance, a pharmacologically active amount of at least one compound of one of the claims 1-3, or a salt thereof, as an active ingredient.

5. A method of preparing pharmaceutical compositions as claimed in claim 4, **characterized in that** a compound of one of the claims 1-3 is brought into a form suitable for administration.

6. A compound as claimed in any of the claims 1-3, or a salt thereof, for use as a medicament

7. Compounds of the general formula (IV) 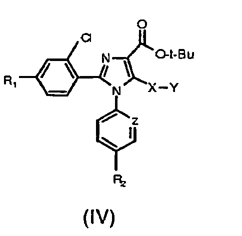 wherein Y represents a methyl, ethyl, n-propyl, isopropyl, n-butyl, t-butyl, trifluoromethyl, phenyl, benzyl or pyridyl group, and R₁, R₂, X and Z have the meanings given in claim 1, such compounds being useful in the synthesis of compounds of the general formula (I).

8. Compounds of the general formula (V) 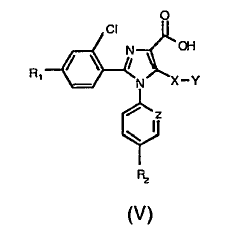 wherein Y represents a methyl, ethyl, n-propyl, isopropyl, n-butyl, t-butyl, trifluoromethyl, phenyl, benzyl or pyridyl group, and R₁, R₂, X and Z have the meanings given in claim 1, such compounds being useful in the synthesis of compounds of the general formula (I).

9. Use of a compound as claimed in any of the claims 1-3 for the preparation of a pharmaceutical composition for the treatment of psychosis, anxiety, depression, attention deficits, memory disorders, cognitive disorders, appetite disorders, obesity, in particular juvenile obesity and drug induced obesity, addiction, appetence, drug dependence and neurological disorders such as neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, and other diseases involving cannabinoid neurotransmission, including the treatment of septic shock, glaucoma, cancer, diabetes, emesis, nausea, asthma, respiratory diseases, gastrointestinal disorders, gastric ulcers, diarrhoea, sexual disorders and cardiovascular disorders.

10. Use of a compound as claimed in any of the claims 1-3 for the preparation of a pharmaceutical composition for the treatment of appetite disorders, in particular obesity, juvenile obesity and drug induced obesity.

11. Use of a compound as claimed in any of the claims 1-3 for the preparation of a pharmaceutical composition for the treatment of eating disorders, in particular obesity, juvenile obesity and drug induced obesity, **characterized in that** said pharmaceutical composition also contains at least one lipase inhibitor.

12. Use as claimed in claim 11, **characterized in that** said lipase inhibitor is orlistat or lipstatin.

## Patentansprüche

1. Verbindungen der allgemeinen Formel (I) 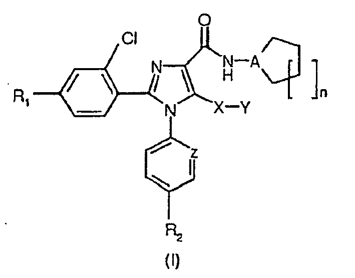 worin
- R₁ ein Chlor-, Brom-, Fluor- oder Wasserstoffatom darstellt,
- R₂ ein Chlor- oder ein Bromatom oder eine CF₃-Gruppe darstellt,
- A ein Stickstoffatom oder eine CH-Gruppe darstellt,
- X ein Schwefelatom oder eine Sulfoxid (S=O) Komponente oder eine Sulfon (SO₂) Komponente darstellt,
- Y ein Wasserstoffatom oder eine Methyl-, Ethyl-, n-Propyl-, Isopropyl-, n-Butyl-, t-Butyl-, Trifluormethyl-, Phenyl-, Benzyl- oder Pyridylgruppe darstellt,
- Z ein Stickstoffatom oder eine CH-Gruppe darstellt,
- n den Wert 1, 2 oder 3 darstellt,
und Tautomere, Stereoisomere und pharmakologisch annehmbare Salze davon.

2. Verbindungen wie in Anspruch 1 beansprucht der allgemeinen Formel (I), worin R₁ ein Wasserstoffatom oder ein Chloratom darstellt, R₂ ein Chloratom darstellt, Y eine Methyl- oder Ethylgruppe darstellt, Z für CH steht, n 1, 2 oder 3 darstellt, A und X die Bedeutungen haben wie in Anspruch 1 gegeben, und Tautomere, Stereoisomere und pharmakologisch annehmbare Salze davon.

3. Verbindung wie in Anspruch 2 beansprucht der allgemeinen Formel (I), worin R₁ ein Chloratom darstellt, Y eine Methylgruppe darstellt, A ein Stickstoffatom darstellt und Tautomere, Stereoisomere und pharmakologisch annehmbare Salze davon.

4. Pharmazeutische Zusammensetzungen, welche zusätzlich zu einem pharmazeutisch annehmbaren Träger und/oder mindestens einer pharmazeutisch annehmbaren Hilfssubstanz eine pharmakologisch wirksame Menge von mindestens einer Verbindung von einem der Ansprüche 1-3 oder ein Salz davon als wirksamen Inhaltsstoff umfassen.

5. Verfahren zum Herstellen von pharmazeutischen Zusammensetzungen wie in Anspruch 4 beansprucht, **dadurch gekennzeichnet, dass** eine Verbindung von einem der Ansprüche 1-3 in eine für Verabreichung geeignete Form gebracht wird.

6. Verbindung wie in einem der Ansprüche 1-3 beansprucht oder ein Salz davon zur Verwendung als Medikament.

7. Verbindungen der allgemeinen Formel (IV) 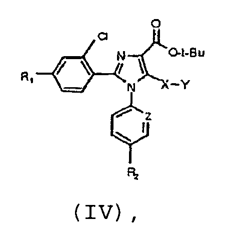 worin Y eine Methyl-, Ethyl-, n-Propyl-, Isopropyl-, n-Butyl-, t-Butyl-, Trifluormethyl-, Phenyl-, Benzyl- oder Pyridylgruppe darstellt und R₁, R₂, X und Z die in Anspruch 1 gegebenen Bedeutungen haben, wobei solche Verbindungen in der Synthese von Verbindungen der allgemeinen Formel (I) brauchbar sind.

8. Verbindungen der allgemeinen Formel (V) 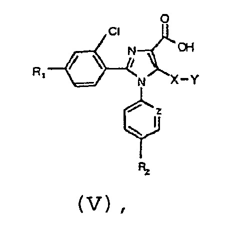 worin Y eine Methyl-, Ethyl-, n-Propyl-, Isopropyl-, n-Butyl-, t-Butyl-, Trifluormethyl-, Phenyl-, Benzyl- oder Pyridylgruppe darstellt und R₁, R₂, X und Z die in Anspruch 1 gegebenen Bedeutungen haben, wobei solche Verbindungen in der Synthese von Verbindungen der allgemeinen Formel (I) brauchbar sind.

9. Verwendung einer Verbindung wie in einem der Ansprüche 1-3 beansprucht für die Herstellung einer pharmazeutischen Zusammensetzung für die Behandlung von Psychose, Angstzuständen, Depression, Aufmerksamkeitsdefiziten, Gedächtnisstörungen, kognitiven Störungen, Appetitstörungen, Fettsucht, insbesondere juveniler Fettsucht und Arznei- bzw. Drogen-induzierter Fettsucht, Sucht, Appetenz, Arznei- bzw. Drogenabhängigkeit und neurologischen Störungen wie neurodegenerativen Störungen, Demenz, Dystonie, Muskelspastizität, Tremor, Epilepsie, Multiple Sklerose, traumatischer Hirnverletzung, Schlaganfall, Parkinson-Krankheit, Alzheimer-Krankheit, Epilepsie, Huntington-Krankheit, Tourette-Syndrom, zerebraler Ischämie, zerebraler Apoplexie, kraniozerebralem Trauma, Schlaganfall, Rückenmarksverletzung, neuroentzündlichen Störungen, Plaque-Sklerose, viraler Enzephalitis, demyelinisierungsbezogenen Störungen, als auch für die Behandlung von Schmerzstörungen, einschließlich neuropathischen Schmerzstörungen und anderen Krankheiten, welche Cannabinoid-Neurotransmission einbeziehen, einschließlich der Behandlung von septischem Schock, Glaukom, Krebs, Diabetes, Erbrechen, Übelkeit, Asthma, Atemwegserkrankungen, gastrointestinalen Störungen, Magengeschwüren, Diarrhoe, sexuellen Störungen und kardiovaskulären Störungen.

10. Verwendung einer Verbindung wie in einem der Ansprüche 1-3 beansprucht für die Herstellung einer pharmazeutischen Zusammensetzung für die Behandlung von Appetitstörungen, insbesondere Fettsucht, juveniler Fettsucht und Arznei- bzw. Drogen-induzierter Fettsucht.

11. Verwendung einer Verbindung wie in einem der Ansprüche 1-3 beansprucht für die Herstellung einer pharmazeutischen Zusammensetzung für die Behandlung von Essstörungen, insbesondere Fettsucht, juveniler Fettsucht und Arznei- bzw. Drogen-induzierter Fettsucht, **dadurch gekennzeichnet, dass** besagte pharmazeutische Zusammensetzung ebenfalls mindestens einen Lipasehemmer enthält.

12. Verwendung wie in Anspruch 11 beansprucht, **dadurch gekennzeichnet, dass** besagter Lipasehemmer Orlistat oder Lipstatin ist.

## Revendications

1. Composés de formule générale (I) 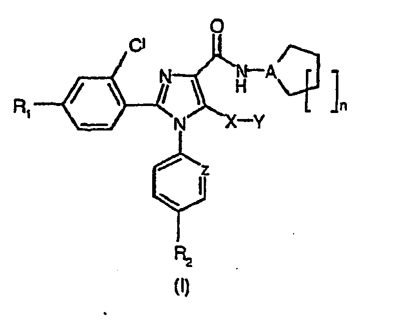 dans laquelle
- R₁ représente un atome de chlore, un atome de brome, un atome de fluor ou un atome d'hydrogène,
- R₂ représente un atome de chlore ou un atome de brome, ou un groupe CF_{3,}
- A représente un atome d'azote ou un groupe CH,
- X représente un atome de soufre ou une fraction sulfoxyde (S=O) ou une fraction sulfone (SO₂),
- Y représente un atome d'hydrogène ou un groupe méthyle, éthyle, n-propyle, isopropyle, n-butyle, t-butyle, trifluorométhyle, phényle, benzyle ou pyridyle,
- Z représente un atome d'azote ou un groupe CH,
- n représente la valeur 1, 2 ou 3,
et des tautomères, des stéréoisomères ainsi que des sels pharmaceutiquement acceptables de ceux-ci.

2. Composés selon la revendication 1 de formule générale (I) dans laquelle R₁ représente un atome d'hydrogène ou un atome de chlore, R₂ est un atome de chlore, Y représente un groupe méthyle ou éthyle, Z est CH, n représente 1, 2 ou 3, A et X ont les mêmes significations que celles données dans la revendication 1, et des tautomères, des stéréoisomères ainsi que des sels pharmaceutiquement acceptables de ceux-ci.

3. Composés selon la revendication 2 de formule générale (I) dans laquelle R₁ représente un atome de chlore, Y représente un groupe méthyle, A est un atome d'azote et des tautomères, des stéréoisomères ainsi que des sels pharmaceutiquement acceptables de celui-ci.

4. Compositions pharmaceutiques comprenant, outre un véhicule pharmaceutiquement acceptable et/ou au moins une substance auxiliaire pharmaceutiquement acceptable, une quantité pharmacologiquement active d'au moins un composé selon l'une des revendications 1 à 3, ou d'un sel de celui-ci, en tant qu'ingrédient actif.

5. Procédé de préparation de compositions pharmaceutiques selon la revendication 4, **caractérisé en ce qu'**un composé selon l'une des revendications 1 à 3 est mis sous une forme appropriée pour une administration.

6. Composé selon l'une quelconque des revendications 1 à 3, ou un sel de celui-ci, pour une utilisation comme médicament.

7. Composés de formule générale (IV) 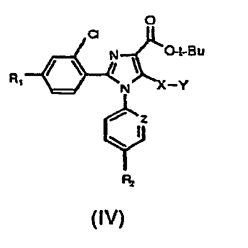 dans laquelle Y représente un groupe méthyle, éthyle, n-propyle, isopropyle, n-butyle, t-butyle, trifluorométhyle, phényle, benzyle ou pyridyle, et R₁, R₂, X et Z ont les mêmes significations que celles données dans la revendication 1, de tels composés étant utiles dans la synthèse de composés de formule générale (I).

8. Composés de formule générale (V) 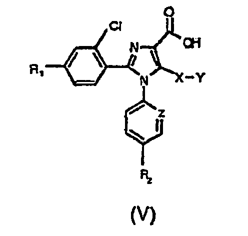 dans laquelle Y représente un groupe méthyle, éthyle, n-propyle, isopropyle, n-butyle, t-butyle, trifluorométhyle, phényle, benzyle ou pyridyle, et R₁, R₂, X et Z ont les mêmes significations que celles données dans la revendication 1, de tels composés étant utiles dans la synthèse de composés de formule générale (I).

9. Utilisation d'un composé selon l'une quelconque des revendications 1 à 3 pour la préparation d'une composition pharmaceutique destinée au traitement de la psychose, de l'anxiété, de la dépression, des troubles déficitaires de l'attention, des troubles de la mémoire, des troubles cognitifs, des troubles de l'appétit, de l'obésité, en particulier l'obésité chez les enfants et l'obésité d'origine médicamenteuse, de l'attachement maladif, de l'appétence, de la pharmacodépendance et des troubles neurologiques tels que les troubles neurodégénératifs, de la démence, de la dystonie, de la spasticité musculaire, du tremblement, de l'épilepsie, de la sclérose en plaques, de la lésion cérébrale traumatique, de l'accident cérébrovasculaire, de la maladie de Parkinson, de la maladie d'Alzheimer, de l'épilepsie, de la maladie de Huntington, du syndrome de Gilles de la Tourette, de l'ischémie cérébrale, de l'apoplexie cérébrale, du traumatisme craniocérébral, de l'accident cérébrovasculaire, de la lésion de la moelle épinière, des troubles neuro-inflammatoires, de la sclérose en plaques, de l'encéphalite virale, des troubles liés à la démyélinisation, de même qu'au traitement des troubles de la douleur, comprenant les troubles de la douleur neuropathique, et d'autres maladies impliquant la neurotransmission de cannabinoïde, comprenant le traitement du choc infectieux, du glaucome, du cancer, du diabète, du vomissement, de la nausée, de l'asthme, des troubles respiratoires, des troubles gastro-intestinaux, des ulcères gastriques, de la diarrhée, des troubles sexuels et des troubles cardiovasculaires.

10. Utilisation d'un composé selon l'une quelconque des revendications 1 à 3, pour la préparation d'une composition pharmaceutique destinée au traitement des troubles de l'appétit, en particulier l'obésité, l'obésité chez les enfants et l'obésité d'origine médicamenteuse.

11. Utilisation d'un composé selon l'une quelconque des revendications 1 à 3, pour la préparation d'une composition pharmaceutique destinée au traitement des troubles de l'alimentation, en particulier l'obésité, l'obésité chez les enfants et l'obésité d'origine médicamenteuse, **caractérisée en ce que** ladite composition pharmaceutique contient également au moins un inhibiteur de lipase.

12. Utilisation selon la revendication 11, **caractérisée en ce que** ledit inhibiteur de lipase est l'orlistat ou la lipstatine.
